# EUROPEAN PATENT SPECIFICATION

(11) **EP 2 950 812 B1**
(45) Date of publication and mention of the grant of the patent: **19.09.2018**
(21) Application number: 14704534.8
(22) Date of filing: 30.01.2014
(51) Int. Cl.: A61K 38/39, A61K 38/17, A61K 31/353, A61K 31/4525, A61K 33/24, A61K 36/67, A61K 33/32, A61P 19/02, A61K 38/01

(54) **COMPOSITIONS COMPRISING PARTIALLY HYDROLYSED HYALURONIC ACID AND CHONDROITIN SULPHATE CONTAINING COLLAGEN, AND SOLUBLE KERATIN**
ZUSAMMENSETZUNGEN ENTHALTEND LÖSLICHES KERATIN UND TEILHYDROLISIERTES KOLLAGEN, DAS HYALURONSÄURE UND CHONDROITINSULFAT ENTHÄLT
COMPOSITIONS COMPRENANT DU COLLAGÈNE PARTIELLEMENT HYDROLYSÉ CONTENANT DE L'ACIDE HYALURONIQUE ET DU SULPHATE DE CHONDROITINE, ET DE LA KÉRATINE SOLUBLE

(30) Priority: 31.01.2013 IT MI20130139
(43) Date of publication of application: 09.12.2015
(73) Proprietor: Sochim International SpA, 20010 Cornaredo (MI) (IT)
(72) Inventor: EIGENMANN, Carlo, I-20149 Milano (MI) (IT); PACCHETTI, Barbara, I-20149 Milano (MI) (IT)
(74) Representative: Villa, Livia
(86) International application number: PCT/EP2014/051789
(87) International publication number: WO 2014/118271

(56) References cited:
- WO-A2-2006/099309
- US-A- 4 906 460
- US-B2- 7 091 180
- N.N.: "BioMada Kundeninfo Arthro Kapseln", , 18 August 2011 (2011-08-18), pages 5-5, XP055071444, Retrieved from the Internet: URL:http://www.biomada.de/BioMada_Kundenin fo_2010.pdf [retrieved on 2013-07-16]
- Anonymous: "Gelatin - Wikipedia", , 30 March 2017 (2017-03-30), XP055360434, Retrieved from the Internet: URL:https://en.wikipedia.org/wiki/Gelatin [retrieved on 2017-03-30]
- Anonymous: "Hydrolyzed collagen - Wikipedia", , 30 March 2017 (2017-03-30), XP055360506, Retrieved from the Internet: URL:https://en.wikipedia.org/wiki/Hydrolyz ed_collagen [retrieved on 2017-03-30]
- SCHAUSS ALEXANDER G ET AL: "Effect of the novel low molecular weight hydrolyzed chicken sternal cartilage extract, BioCell Collagen, on improving osteoarthritis-related symptoms: a randomized, double-blind, placebo-controlled trial.", JOURNAL OF AGRICULTURAL AND FOOD CHEMISTRY 25 APR 2012, vol. 60, no. 16, 25 April 2012 (2012-04-25), pages 4096-4101, ISSN: 1520-5118

## Description

### Summary of the invention

The present invention relates to compositions for oral administration which have a beneficial effect on cartilage disease, osteoarthritis (OA) and joint disease with an inflammatory component and contain, as active ingredients, a collagen matrix rich in hyaluronic acid and chondroitin sulphate, a soluble keratin and optionally manganese, preferably combined with piperine extract and/or catechin extract or epigallocatechin gallate.

### Technical background

Cartilage disease affects the cartilage tissue around the bone, and takes the form of a degenerative process of the cartilage. Cartilage diseases of the joints are due to metabolic or degenerative causes; the latter are isolated, or associated with joint cartilage disease, and are due to congenital metabolic alterations (chondrodysplasia).

Cartilage disease can be due to aging, or to wear caused by excessive use of the joint by an individual.

Three grades of cartilage disease are usually identified:
1) softening of the cartilage without fissuring;
2) local or diffuse fissuring;
3) loss of cartilage ground substance with exposure of the bone.

Cartilage lesions result from traumas, such as sprains, falls, fractures or excessive joint use, which can cause focal damage to joint cartilage that was not necessarily already affected by degenerative processes.

Cartilage wear is chronic and irreversible. It tends to worsen with time, at differing rates, causing pain symptoms of varying severity.

In this case, pharmacological treatment is often symptomatic, traditionally involving the use of painkillers such as paracetamol or NSAIDs (non-steroidal anti-inflammatory drugs) which, as well as alleviating pain, counteract the inflammatory processes that take place episodically or constantly in a joint with worn cartilage.

With time, cartilage disease degenerates to osteoarthritis.

Osteoarthritis (OA) is a chronic disorder characterised by degeneration and inflammation of the joint tissue. From the clinical standpoint, patients with OA experience pain, stiffness and reduced joint movement. The progress of the disease is often aggressive, leading to disability. OA is the most prevalent of the various joint diseases, and has the highest socioeconomic impact worldwide. (Peat G, et al. Ann Rheum Dis 2001).

Its incidence increases with age. It is one of the most common causes of painful joint disease (Felson DT, et al. Arthritis Rheum 1987).

Elderly patients present various comorbidities which add to the complexity of pharmacological treatment.

During the manifestation of this disorder, new connective tissue and new bone form around the affected area. The affected joint presents characteristic alterations of the cartilage, with thinning, fissuring, formation of marginal osteophytes, and zones of subchondral osteosclerosis in the load-bearing areas. All the symptoms and clinical signs presented are located in the affected joint, namely pain, limited movement, stiffness and joint deformity.

No cure for OA has yet been found.

The available drugs only aim to reduce the pain symptoms, and the majority give rise to side effects which may be serious (paracetamol and NSAIDs). Other compounds are used by oral administration to treat osteoarthritis, such as glucosamine, or combinations of glucosamine and chondroitin sulphate, glucosamine and chondroitin and collagen.

Infiltration treatment with medicaments based on hyaluronic acid with different molecular weights is available in addition to oral treatment (Richette P et al. (March 2009), Arthritis Rheum. 60 (3): 824-30.). Hyaluronic acids are mainly used to treat cartilage disease of the knee, hip and ankle, injected into the joint to produce viscosupplementation, thus increasing the lubrication of the joint.

Although the pathogenetic mechanisms of osteoarthritis are becoming increasingly clear, no medicaments have yet been developed which have demonstrated a definite ability to slow the progress of the disease. The efficacy of chondroprotectants and SYSADOA (SYmptomatic Slow-Acting Drugs for OsteoArthritis) is still controversial.

Various guidelines, such as those of the European League Against Rheumatism (EULAR), recommend both pharmacological and non-pharmacological approaches. There is consequently a need for new therapeutic options for the treatment of OA.

The identification of new substances that offer benefits or modulate the onset and severity of the disease may have important health implications, and remains the main challenge for this disease.

It was recently found that hyaluronic acid, when administered orally, reduces joint stiffness and pain in patients suffering from OA, and its efficacy is supported by clinical evidence. The latest evidence relates to the use of a hydrolysed collagen matrix with low molecular weight and a high content of hyaluronic acid and depolymerised chondroitin sulphate. A randomised, double-blind, placebo-controlled trial demonstrated the efficacy of hyaluronic acid (HA) and depolymerised chondroitin sulphate (CS), in a collagen matrix, on the symptoms of OA. The results confirm a significant reduction in pain on the VAS scale, and a significant reduction in the WOMAC score, after 70 days' treatment, demonstrating that it is effective in treating the symptoms associated with OA, therefore improving patients' quality of life (Schauss A.G. et al., J. Agric. Food Chem. 60: 4096-4101).

Corpsa, A.N., et al. (Matrix Biology 23 (2004) 163-169) described the inhibiting effects of epigallocatechin gallate (EGCG) on the expression of collagenase, matrix metalloproteinase (MMP) and stromelysin (MMP-3) induced by interleukin-lb in human tendon fibroblasts, suggesting that breakdown of the extracellular matrix may constitute a potential therapeutic target for the action of green tea polyphenols.

### Description of the invention

It has now been found that by combining a soluble keratin with partly hydrolysed collagen, synergic effects useful for the treatment of cartilage disease, osteoarthritis and joint disease are obtained. In addition to their synergic effects, especially on inflammation, the nutraceutical, dietary and nutritional compositions according to the invention induce surprisingly rapid analgesic effects, allowing rapid, effective treatment of the symptoms of cartilage disease, osteoarthritis (primary and secondary), joint disease with an anti-inflammatory component, and the associated pain syndromes.

The object of the invention is therefore oral compositions comprising:
a) partly hydrolysed collagen containing at least 10% hyaluronic acid and at least 20% chondroitin sulphate, and
b) a soluble keratin.

Partly hydrolysed collagen containing at least 10% hyaluronic acid and at least 20% chondroitin sulphate is available on the market, and is known from US 6 025 327, US 6 323 319, US 6 780 841 and US 7 091 180.

Hyaluronic acid and chondroitin sulphate of different origins could be used as an alternative to or combined with said ingredient, provided that they are capable of being absorbed after oral administration.

The soluble keratin is preferably an S-sulphonated keratin as described in WO 03/011894, WO 2006/099309 and WO 2009/059161.

The compositions according to the invention may also contain one or more of the following ingredients:
c) piperine,
d) manganese, and
e) catechin extract or epigallocatechin gallate.

The compositions preferably contain the active components within the following weight ranges per unit dose:
a) partly hydrolysed collagen, in amounts ranging from 250 mg to 1500 mg,
b) soluble keratin, in amounts ranging from 100 to 500 mg,
c) piperine, in amounts ranging from 0.5 mg to 5 mg,
d) manganese, in amounts ranging from 0.3 mg to 10 mg.

Catechin extract or epigallocatechin gallate obtained from green tea, titrated to at least 90%, can optionally be present in amounts ranging from 25 mg to 300 mg.

The compositions are preferably in the form of tablets, capsules, soft gels, orodispersible granular powders or powders for extempore use and liquid preparations, suitable for oral administration, to be administered one, two or three times a day.

The compositions according to the present invention could be formulated suitably for oral administration, and will be prepared according to conventional methods well known in pharmaceutical technology, using excipients, diluents, fillers and other carriers acceptable for their final use.

A composition example is set out below.

| **COMPOSITION** | **PER TABLET** | **% RDA* PER TABLET** |
|---|---|---|
| Collagen matrix | 1200 mg | - |
| - with 10% hyaluronic acid | - 120 mg | |
| - with 20% chondroitin sulphate | - 240 mg | |
| Soluble keratin | 300 mg | - |
| Piperine from pepper, 95% extract | 2.5 mg | - |
| Manganese | 10 mg | 500% |

| | | |
|---|---|---|
| *RDA = Recommended Dietary Allowance | | |

## Claims

1. Compositions comprising:
a) partially hydrolysed collagen containing at least 10% hyaluronic acid and at least 20% chondroitin sulphate,
b) a soluble keratin,
and optionally one or more of the following ingredients:
c) piperine,
d) manganese,
e) catechin extract or epigallocatechin gallate.

2. Compositions according to claim 1 wherein the soluble keratin is an S-sulphonated keratin.

3. Compositions according to claim 1 or 2, comprising the active ingredients within the following ranges by weight per unit dose:
a) partly hydrolysed collagen, in amounts ranging from 250 mg to 1500 mg,
b) soluble keratin, in amounts ranging from 100 to 500 mg,
c) piperine, in amounts ranging from 0.5 mg to 5 mg,
d) manganese, in amounts ranging from 0.3 mg to 10 mg.

4. Compositions according to one or more of claims 1 to 3, also containing catechin extract or epigallocatechin gallate.

5. Compositions according to claim 4, containing catechin extract or epigallocatechin gallate in amounts ranging from 25 mg to 300 mg.

6. Compositions according to the above claims, for use in the treatment of cartilage disease, osteoarthritis and joint disease.

## Patentansprüche

1. Zusammensetzungen, die Folgendes umfassen:
a) teilweise hydrolysiertes Kollagen, das wenigstens 10 % Hyaluronsäure und wenigstens 20 % Chondriotinsulfat enthält,
b) ein lösliches Keratin, und optional einen oder mehrere der folgenden Inhaltsstoffe:
c) Piperin,
d) Mangan,
e) Catechin-Extrakt oder Epigallocatechingallat.

2. Zusammensetzungen nach Anspruch 1, wobei das lösliche Keratin ein S-sulfoniertes Keratin ist.

3. Zusammensetzungen nach Anspruch 1 oder 2, umfassend die aktiven Inhaltsstoffe innerhalb der folgenden Bereiche nach Gewicht pro Dosierungseinheit:
a) teilweise hydrolysiertes Kollagen, in Mengen im Bereich von 250 mg bis 1500 mg,
b) lösliches Keratin, in Mengen im Bereich von 100 bis 500 mg,
c) Piperin, in Mengen im Bereich von 0,5 mg bis 5 mg,
d) Mangan, in Mengen im Bereich von 0,3 mg bis 10 mg.

4. Zusammensetzungen nach einem der Ansprüche 1 bis 3, ebenfalls enthaltend Catechin-Extrakt oder Epigallocatechingallat.

5. Zusammensetzungen nach Anspruch 4, enthaltend Catechin-Extrakt oder Epigallocatechingallat in Mengen im Bereich von 25 mg bis 300 mg.

6. Zusammensetzungen nach einem der vorhergehenden Ansprüche zur Verwendung in der Behandlung von Knorpelerkrankungen, Osteoarthritis und Gelenkerkrankungen.

## Revendications

1. Compositions comprenant :
a) du collagène partiellement hydrolysé contenant au moins 10 % d'acide hyaluronique et au moins 20 % de sulfate de chondroïtine,
b) une kératine soluble, et éventuellement un ou plusieurs des ingrédients suivants :
c) pipérine,
d) manganèse,
e) un extrait de catéchine ou du gallate d'épigallocatéchine.

2. Compositions selon la revendication 1, dans lesquelles la kératine soluble est une kératine S-sulfonée.

3. Compositions selon la revendication 1 ou 2, comprenant les ingrédients actifs dans les plages suivantes en poids par dose unitaire :
a) collagène partiellement hydrolysé, en quantités variant de 250 mg à 1500 mg,
b) kératine soluble, en quantités variant de 100 à 500 mg,
c) pipérine, en quantités variant de 0,5 mg à 5 mg,
d) manganèse, en quantités variant de 0,3 mg à 10 mg.

4. Compositions selon l'une ou plusieurs des revendications 1 à 3, contenant également un extrait de catéchine ou du gallate d'épigallocatéchine.

5. Compositions selon la revendication 4, contenant un extrait de catéchine ou du gallate d'épigallocatéchine en quantités variant de 25 mg à 300 mg.

6. Compositions selon les revendications ci-dessus, destinées à être utilisées dans le traitement d'une maladie du cartilage, de l'arthrose et d'une maladie articulaire.
